# EUROPEAN PATENT SPECIFICATION

(11) **EP 2 809 238 B1**
(45) Date of publication and mention of the grant of the patent: **18.01.2017**
(21) Application number: 13705645.3
(22) Date of filing: 31.01.2013
(51) Int. Cl.: A61B 17/00, A61B 17/29

(54) **MEDICAL DEVICE HAVING A MODULAR CONTROLLER**
MEDIZINISCHE VORRICHTUNG MIT EINEM MODULAREN REGLER
DISPOSITIF MÉDICAL AYANT UN CONTRÔLEUR MODULAIRE

(30) Priority: 31.01.2012 US 201261593209 P
(43) Date of publication of application: 10.12.2014
(73) Proprietor: Boston Scientific Scimed, Inc., Maple Grove, MN 55311-1566 (US)
(72) Inventor: GOLDEN, John, Norton, MA 02766 (US); STANTON, Larry, Burlington, MA 01803 (US); WOOD, Mark, Shrewsbury, MA 01545 (US); DAYTON, Peter, Brookline, MA 02445 (US); DURR, Bernadette, Marlborough, MA 01752 (US); ZALEWSKI, Brandon, Clinton, MA 01510 (US); SEDDON, Dane, Boston, MA 02127 (US); KEENE, Kenneth, Winchester, MA 01890 (US)
(74) Representative: Vossius & Partner Patentanwälte Rechtsanwälte mbB
(86) International application number: PCT/US2013/024113
(87) International publication number: WO 2013/116499

(56) References cited:
- WO-A1-2007/002545
- WO-A1-2008/070556
- US-A1- 2011 172 706

## Description

### DESCRIPTION OF THE INVENTION

### Field of the Invention

Embodiments of the invention include medical devices, and more particularly, a medical device having a modular controller.

### Background of the Invention

CN 101 594 816 A discloses various systems and methods for facilitating control of a tool or tools. The systems can allow a user to control multiple degrees of freedom. One such system allows a user to control multiple degrees of freedom of two tools simultaneously. Another such system allows a user to control multiple degrees of freedom with a single hand. Frames and rails for supporting and/or constraining movement of a tool or tools are also described.

Minimally invasive surgical instruments, such as endoscopic and laparoscopic devices, provide access to surgical sites while minimizing patient trauma. The growing capabilities of such therapeutic devices allow physicians to perform an increasing variety of surgeries through minimally invasive routes. This increasing variety of surgeries presents new challenges for device manufacturers because a greater variety of instruments is required.

Producing a large range of different devices can be complicated and expensive. Each type of device may require a different type of controller to control the functions unique to that specific device. Different controllers may require their own configuration, moving parts, assembly steps, molds, etc. In addition, controllers having right-handed and left-handed orientations may require different manufacturing processes. Surgeons may also wish to change the configuration of a given controller during a surgery without removing and replacing the entire instrument.

As described herein, one aspect of the present disclosure is directed to a modular medical device. Such a device could include a common device chassis configurable to receive various replaceable elements. The replaceable elements could be interchangeable with other elements and shared across a range of devices. For example, a modular controller could be adapted for use with a grasper or a dissector. A controller configured for use with the grasper could include a ratcheting mechanism to selectively lock jaw movement. The same controller chassis could also be configured for use with a dissector that may lack the ratcheting mechanism. Costs could be reduced by using common and replaceable elements to produce different types of devices configured for specific uses.

Modular devices may also improve the safety of surgical procedures involving the application of electrical or other forms of energy. Inadvertent contact between "cold" and "hot" instruments can shock a patient or a surgeon. Some endoscopic procedures increase the risk of shocking because of the proximity of instruments over longer distances compared to laparoscopic procedures. Capacitive coupling can build up dangerous voltages when two conductors are separated by an insulator. Stray electrical current may travel along a cold instrument to the surgeon or discharge to the patient without the surgeon's knowledge. Electrical safety can be further compromised because endoscopic instrument shafts are often subjected to high bending forces and strains that can increase the likelihood of insulation breakdown.

The medical devices described herein aim to overcome these and other limitations of the prior art.

### SUMMARY OF THE INVENTION

The invention is defined by the features of the independent claim. Preferred embodiments of the invention are recited in the dependent claims. One aspect of this invention is directed to a surgical instrument having an elongate shaft with a proximal and a distal end, and including a controller coupled to a proximal region of the elongate shaft. In a first configuration, the controller can control an end-effector coupled to the distal end of the elongate shaft via a control element moveably coupled to the controller. In a second configuration, the controller can move the distal end of the elongate shaft. The surgical instrument can also have an aperture configured to receive the control element in the first configuration and receive an insert element fixedly coupled to the controller and configured to cover the aperture in the second configuration.

The disclosure is also directed to a controller for an instrument. The controller can include a base coupled to a proximal region of an elongate shaft of the instrument and a handle movably coupled to the base. The handle can further comprise an elongate control member formed of conductive material, moveably coupled to the handle, and moveable to control a distal end of the elongate shaft. The handle can also include an aperture configured to receive a control element moveably coupled to the handle. The control element can also be replaceable with an insert element configured to at least partially cover the aperture.

The disclosure also relates to a surgical instrument for electrically safe use by an operator. The instrument can comprise an elongate shaft including a proximal, a distal end, and a conductive member. The instrument can also comprise a controller coupled to a proximal region of the elongate shaft. Part of the conductive member can be located within the controller. The controller can be formed of non-conductive material. The instrument can further include at least one aperture configured to receive a control element in a first operational configuration and an insert element in a second operational configuration. The control element and the insert element can be formed of non-conductive material. The at least one aperture can be substantially covered to prevent discharge of an operational current from the conductive member to the operator.

Another aspect of the disclosure is directed to a method of assembling a first type of a medical device and a second type of medical device different to the first type of medical device. The method can include forming a plurality of surgical chassis. The method can then include coupling a control element to a first of the plurality of surgical chassis to form the first type of medical device. The control element can be moveably coupled to the first of the plurality of surgical chassis and configured to move relative to the first of the plurality of surgical cassis to control a function of the first type of medical device. The method can further include coupling an insert element to a second of the plurality of surgical chassis to form a second type of medical device. The insert element can be fixedly coupled to the second of the plurality of surgical chassis and configured to form at least part of a body of the second type of medical device.

Additional objects and advantages of the invention will be set forth in part in the description which follows, and in part will be obvious from the description, or may be learned by practice of the invention. The objects and advantages of the invention will be realized and attained by means of the elements and combinations particularly pointed out below.

It is to be understood that both the foregoing general description and the following detailed description are exemplary and explanatory only and are not restrictive of the invention.

### BRIEF DESCRIPTION OF THE DRAWINGS

The accompanying drawings, which are incorporated in and constitute a part of this specification, illustrate embodiments of the invention and together with the description, serve to explain the principles of the invention.
Figure 1 is a perspective view of a device, according to an exemplary embodiment of the invention;
Figure 2 is a perspective view of a system including the device, according to an exemplary embodiment of the invention;
Figure 3 is a perspective view of three end-effectors, according to exemplary embodiments of the invention;
Figure 4A is a perspective view of a shaft, according to an exemplary embodiment of the invention;
Figure 4B is a close-up top view of a handle, according to an exemplary embodiment of the invention;
Figure 5 is a cut-away view of a handle and a perspective view of a shaft, according to an exemplary embodiment of the invention;
Figure 6A is a top view of a handle, according to an exemplary embodiment of the invention;
Figure 6B is a cut-away top view of the handle, as shown in FIG. 6A;
Figure 7A is a perspective view of a handle, according to an exemplary embodiment of the invention;
Figure 7B is a cut-away top view of the handle, as shown in FIG. 7A;
Figure 7C is an exploded perspective view of the handle, as shown in FIG. 7A;
Figure 8A is a perspective view of a handle, according to another exemplary embodiment of the invention;
Figure 8B is a cut-away top view of the handle, as shown in FIG. 8A;
Figure 8C is an exploded perspective view of the handle, as shown in FIG. 8A;
Figure 9A is a perspective view of a handle, according to another exemplary embodiment of the invention;
Figure 9B is a cut-away top view of the handle, as shown in FIG. 9A;
Figure 9C is an exploded perspective view of the handle, as shown in FIG. 9A;
Figure 10 is a cut-away side view of instruments in a guide tube, according to an exemplary embodiment of the invention;
Figure 11A is a perspective view of a cable, according to an exemplary embodiment of the invention;
Figure 11B is a perspective view of a cable, according to another exemplary embodiment of the invention;
Figure 11C is a cut-away perspective view of the cable, as shown in FIG. 11B;
Figure 12A is a perspective view of a cable, according to an exemplary embodiment of the invention;
Figure 12B is a perspective view of a cable, according to another exemplary embodiment of the invention;
Figure 12C is a cut-away perspective view of the cable, as shown in FIG. 12B; and
Figure 13 is a cut-away side view of a seal, according to an exemplary embodiment of the invention.

### DESCRIPTION OF THE EMBODIMENTS

Reference will now be made in detail to exemplary embodiments of the invention, examples of which are illustrated in the accompanying drawings. Wherever possible, the same reference numbers will be used throughout the drawings to refer to the same or like parts. Also, any aspect set forth in any embodiment may be used with any other embodiment set forth herein.

While the discussion of systems and methods may generally refer to "surgical tools," "surgery," or a "surgical site" for convenience, the described systems and their methods of use are not limited to tissue resection or repair. In particular, the described systems and devices can be used for inspection and diagnosis in addition, or as an alternative to surgery. Moreover, the systems and devices described herein can perform non-medical applications such as in the inspection or repair of machinery.

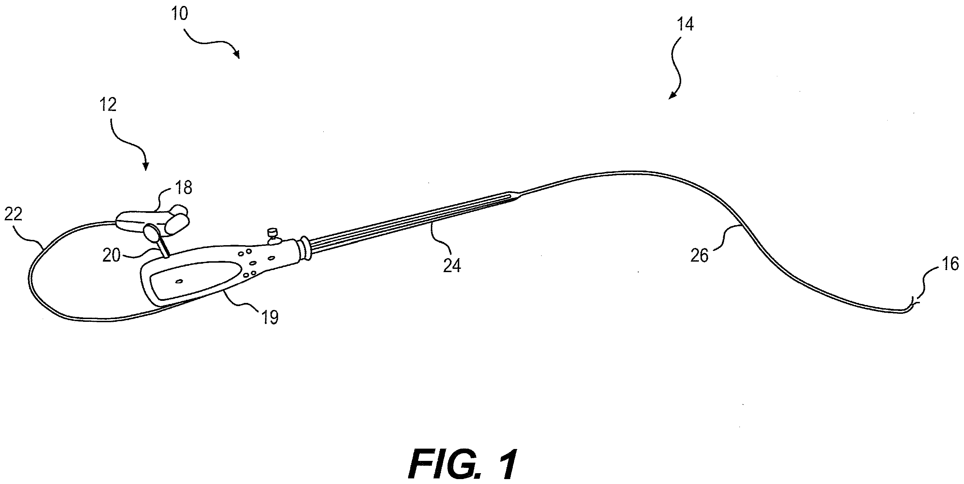

FIG. 1 depicts a device 10, according to an exemplary embodiment. Device 10 can include a medical device configured for use with a surgical method, including a therapeutic or a diagnostic procedure. For example, device 10 can be configured for use with an endoscope, a guide tube, an access catheter, or other type of device configured to access a patient's body. Device 10 may be used for procedures within or adjacent to various body organs, such as, an esophagus, a heart, a stomach, a pelvic area, a bladder, an intestine, or any other portion of a gastrointestinal, urinary, or pulmonary tract.

Device 10 may be configured for insertion into a patient's body through an anatomical opening. In other embodiments, device 10 may be used in natural orifice transluminal endoscopic surgery (NOTES) procedures or single incision laparoscopic surgical (SILS) procedures. Accordingly, device 10 can be shaped and sized for placement into a patient via a body cavity or an incision.

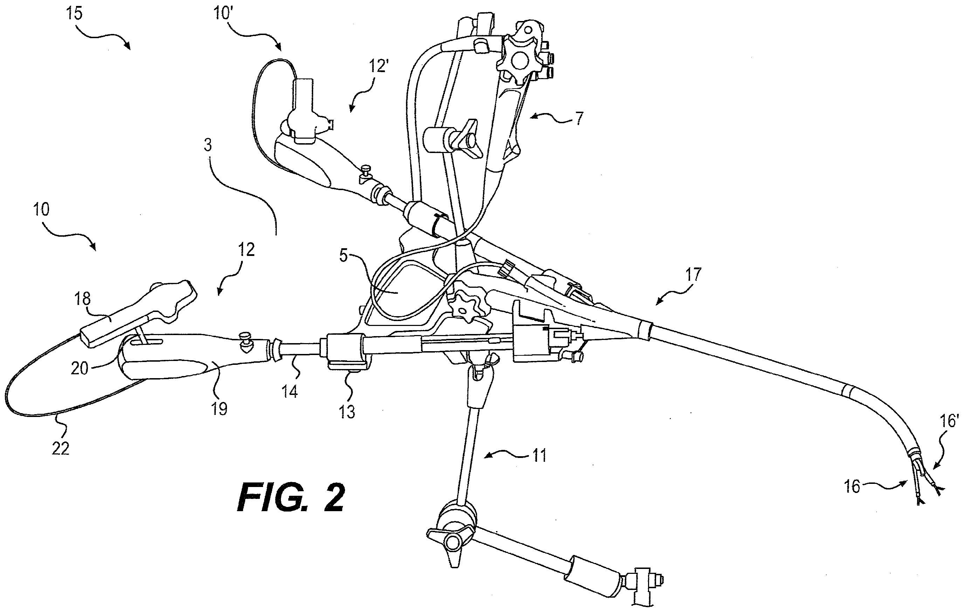

As shown in FIG. 2, device 10 can be used as part of a system 15. For example, device 10 can be moveably coupled to a frame 11 to permit movement of device 10 relative to a patient (not shown). Frame 11 can be coupled to a guide tube 17, part of which can be located within the patient. System 15 can also include an optical system, such as an endoscope 7. An exemplary system is described in U.S. Patent Application Publication No. 2008/0188868.

In operation, when used in conjunction with system 15, a user can grasp a first controller 12 with a first hand and a second controller 12' with a second hand, as shown in FIG. 2. The user can move controllers 12, 12' forward and backwards, or rotate them relative to frame 11 to control forward, backward, and rotational movement of distal end-effectors 16, 16'. A user can also rotate controllers 12, 12' clockwise and anticlockwise relative to frame 11 to articulate distal end-effectors 16, 16' left and right. Tilting controllers 12, 12' forward and backwards relative to frame 11 can articulate distal end-effectors 16, 16' up and down. Articulation of distal end-effectors 16, 16' in these four degrees of freedom can be achieved simultaneously and without the user losing contact with controllers 12, 12'. Controllers 12, 12' can also actuate distal end-effectors 16, 16' to open and close them to provide a fifth degree of freedom. Furthermore, controllers 12, 12' can be configured for left-handed or for right-handed operation. In FIG. 2, ambidextrous device 10 is configured for right-handed operation, and ambidextrous device 10' is configured for left-handed operation.

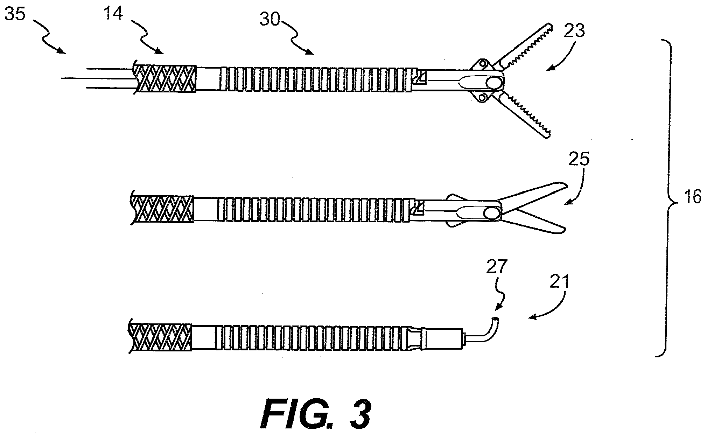

Device 10 can also be configured for use with a variety of different end-effectors 16, examples of which are shown in FIG. 3. These and other types of end-effectors may be distally located on shaft 14 and can be articulated or actuated by controller 12 (shown in FIG. 1). One or more control members 35 of device 10 can be moved to control articulation of steering section 30. One or more different control members 35 in device 10 can be moved to control actuation of end-effectors 16.

Device 10 can be configured to mechanically transmit movement or forces from controller 12 to elongate shaft 14 via one or more control members 35, shown in FIG. 3. At least part of one or more control members 35 may extend through controller 12 or elongate shaft 14. Control members 35 may include one or more articulation members that can be moved to articulate one or more parts of elongate shaft 14, such as, for example, steering section 30. Control members 35 may include one or more actuation members that can be moved to actuate one or more end-effectors 16 on elongate shaft 14, such as, for example, to open and close jaw members of a grasper or a dissector.

Control members 35 can include a cable, a wire, a ribbon, a rod, a Bowden cable, or other type of elongate element configured to transfer a movement or a mechanical force. For example, control members 35 could include a metal alloy, braided synthetic thread, polymer, coil, or similar flexible material configured to transfer a tensile force. A Bowden cable, for instance, can include an inner member configured to transfer tensile force and an outer member configured to transfer compressive force. In some embodiments, the outer member can include an incompressible helical coil.

Control members 35 can be configured to transfer tensile force, compressive force, or both tensile and compressive forces. Control members 35 may also include one or more types of elongate element and may be mechanically coupled to one or more gears, pulleys, capstans, or other mechanical devices configured to transfer mechanical forces or movement. Further, control members 35 may be sized and shaped depending on load requirements and geometric constraints.

In FIG. 3, a dissector 23 and a pair of scissors 25 can be provided. Both dissector 23 and scissors 25 can include multiple control members 35 configured to provide articulation and actuation of both types of end-effectors 16. End-effectors 16 could also include an electrosurgical hook 21, having a hook 27 configured to apply ablative energy to tissue. Electrosurgical hook 21 may require articulation and may not require actuation. Another end-effector (not shown) could include a bent tube configured to provide suction or irrigation to a distal region of instrument 10. This tube may not require articulation or actuation.

In some embodiments device 10 can be modular, wherein one or more elements of device 10 can be substituted with one or more other elements during manufacture. Such a modular device 10 can be used to manufacture a range of different types of devices 10 depending on the type or function of device 10. Device 10 could include a common chassis forming part of a frame or structure of device 10.

Various interchangeable elements could be configured to function with the common chassis. For example, if device 10 includes end-effector 16 that requires articulation and actuation, controller 12 can include control elements configured to provide articulation and actuation. If device 10 requires only articulation, the actuation control elements of controller 12 can be replaced with insert elements. If device 10 includes electrosurgical hook 21, various elements of controller 12 can be insulative to protect the patient or surgeon from unintentional shocks. The same controller 12 can also be configured for left-handed or right-handed operation by the surgeon.

In some embodiments, controller 12 can comprise a handle 18 moveably coupled to a base 19. Handle 18 may be ambidextrous and may detach from shaft 20 to permit a change in handedness. For example, handle 18 can detach from shaft 20 and flip upside-down to switch from a left-handed to a right-handed configuration. Various exemplary handles are described in U.S. Patent Application Publication No. 2008/0287862.

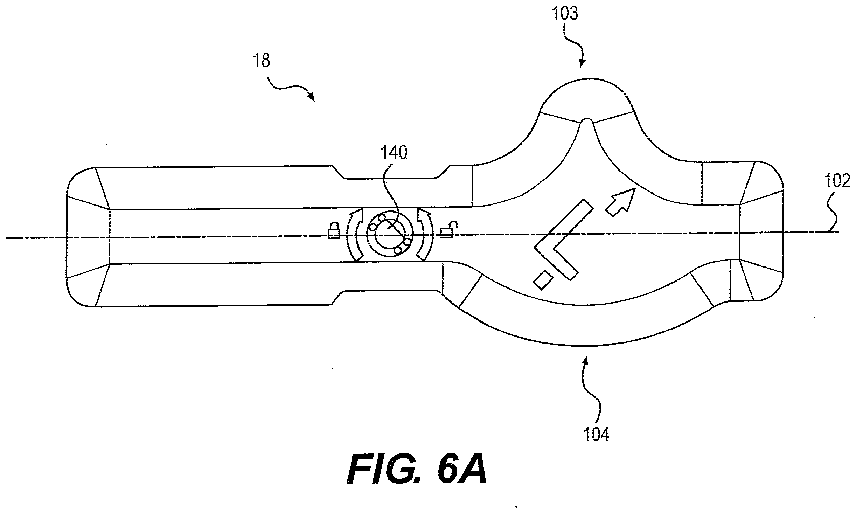

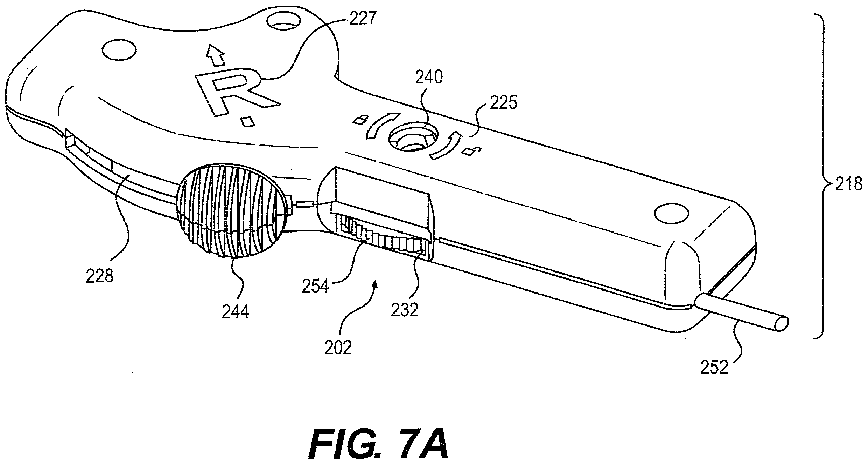

FIGS. 6A-B show handle 18 from FIG. 1 with a common handle chassis 118 as it would appear before assembly. Chassis 118 contains no control elements and no insert elements. One or more control elements or insert elements can be added to chassis 118 to form handles 218, 318, 418 shown in FIGS. 7A-9C.

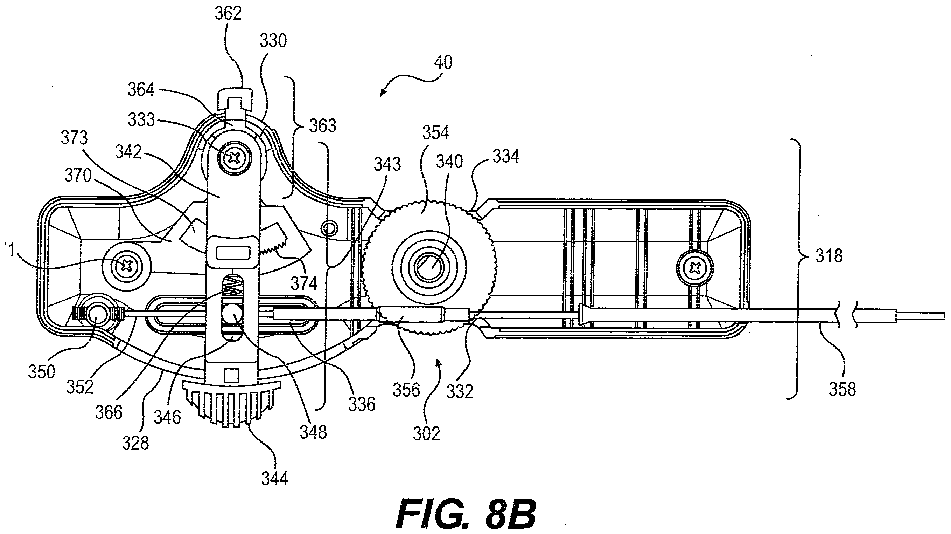

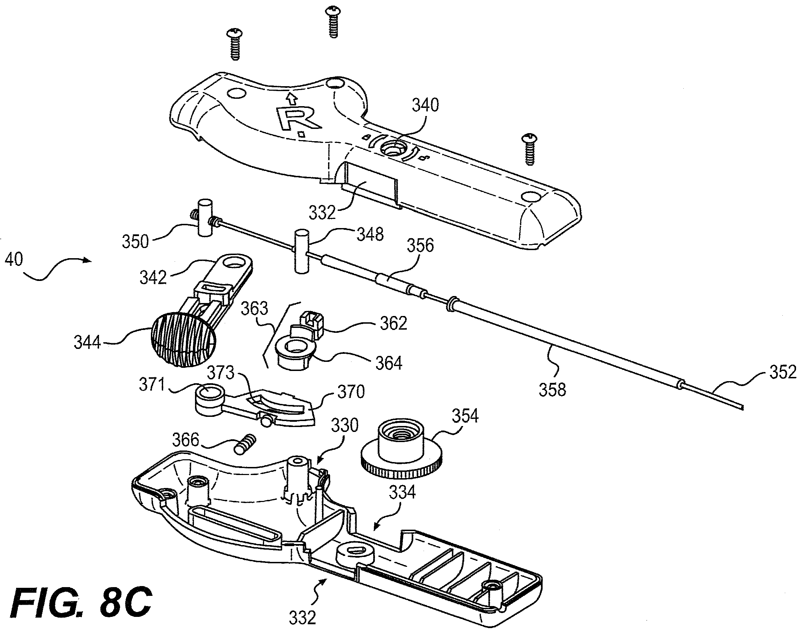

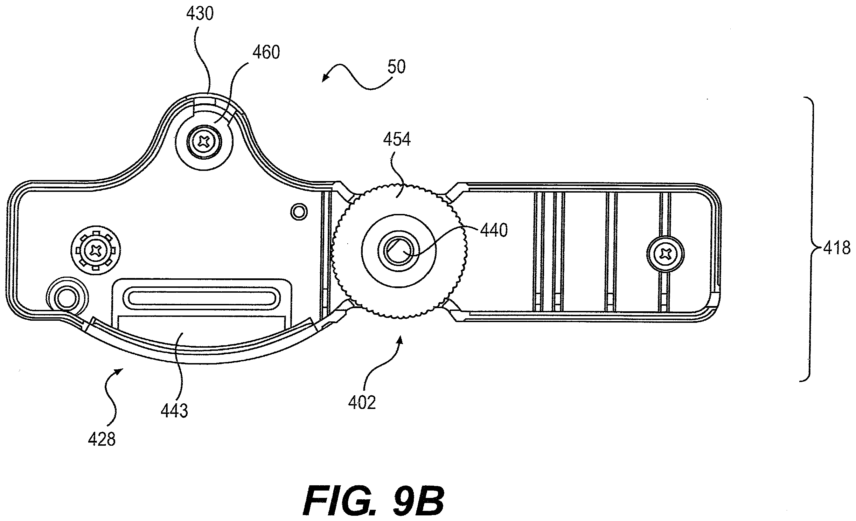

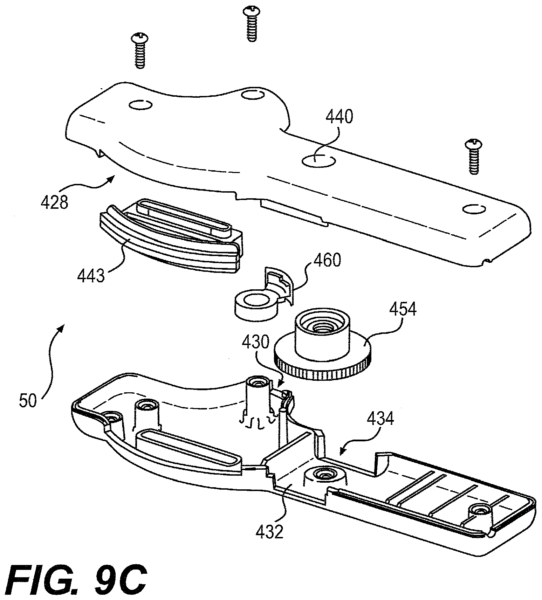

As described below, control elements 40 (shown in FIGS. 8B, 8C) and insert elements 50 (shown in FIGS. 9B, 9C) can include various moveable or stationary components configured to operate with chassis 118. For instance, control elements 40 could include a thumb wheel, lever, pull ring, handle, button, slide, turn-key, ratchet, gear, trigger, or switch. Control elements 40 may be moveably coupled to chassis 118 via a structure to control a function of device 10. For instance, control elements 40 can be configured to move or control a distal region of shaft 14 or distal end effector 16. Control elements 40 may also be configured to control attachment of handle 18 relative to base 19. Control elements 40 may also include other components configured to control a function of device 10.

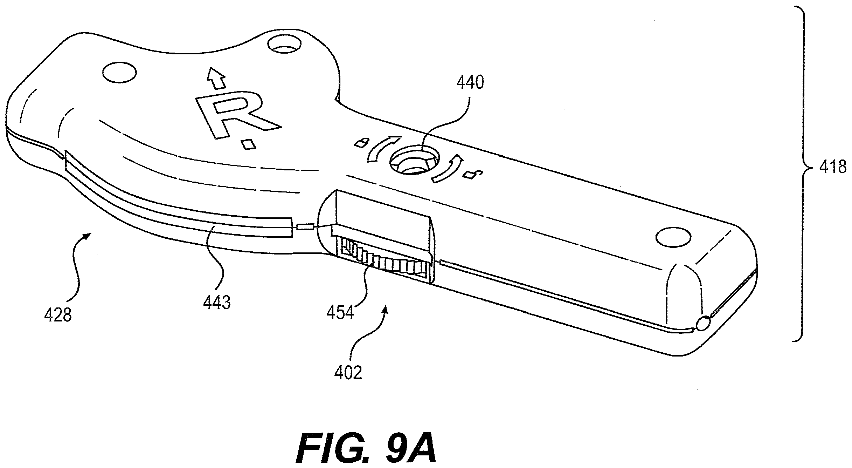

Insert elements 50 could include a stationary component fixedly attached to chassis 118. Insert element 50 may include a flange or other structure configured to fixedly attach insert element 50 to chassis 118. Insert element 50 may lie flush with handle 418, as is shown in FIGS. 9A-9C, or may protrude from aperture 428 to take any shape. Insert element 50 could function to at least partially seal apertures 428, 430 in handle 418. For instance, handle 418 could include insert element 50 molded to provide hand or finger support, a hook or latch to create other useful configurations for gripping handle 418, or may serve any purpose other than controlling a function of handle 418. Insert element 50 could also include an outlet cover for an electrical adapter, a fluid port, or a grounding wire.

In some embodiments, device 10 can include a modular controller 12 with one or more apertures configured to receive different control elements 40 or insert elements 50. In one embodiment, handle 18 can include at least one aperture located on at least one face of handle 18. An aperture or opening may extend at least partially across a structure or be configured to receive one or more control elements 40 and insert elements 50. Other apertures or openings in device 10 can be variously positioned about device 10. For example, an aperture could be configured to receive a connector, a cable, or other component passing into or out of part of device 10.

Engaging and disengaging handle 18 can comprise, for example, a variety of mechanical or frictional mating features. As shown in FIG. 6A, the mating feature can include an opening 140 in chassis 118 for receiving a portion of shaft 20 (not shown). Opening 140 can extend from a first face of chassis 118 to an opposite second face. In another embodiment, two mating features can be differently located on the different sides of chassis 118. Chassis 118 may also snap-fit onto shaft 20. Additionally, chassis 118 can be held in place by a number of different attachment mechanisms 202 like those shown in FIG. 7A, for instance, a thumb screw 254.

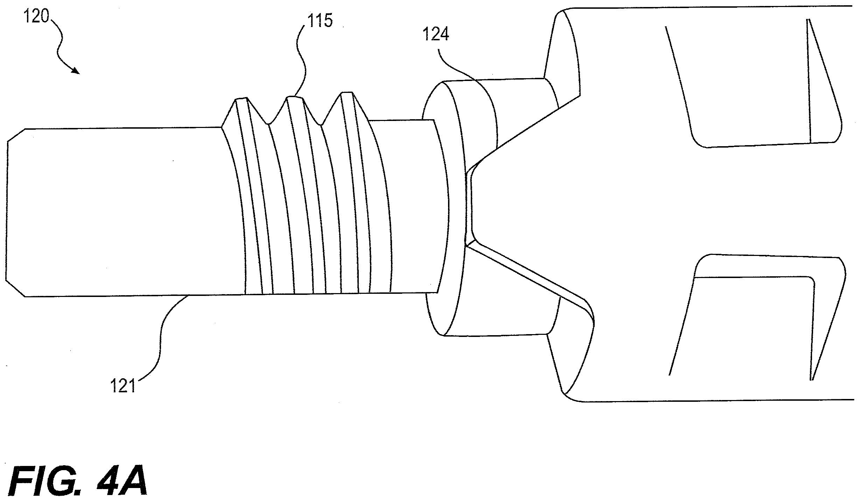

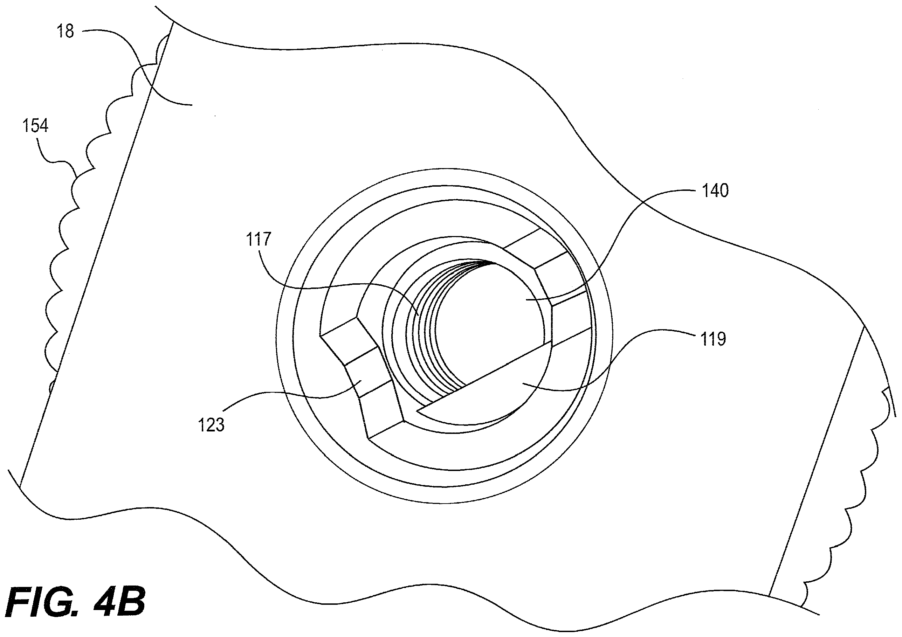

In some embodiments, handle 18 in FIG. 1 may be configured to mate with shaft 20 so that handle 18 locks into place on shaft 20 at one set position relative to base 19. To assist with positioning handle 18 in the correct orientation relative to base 19, handle 18 may include a self-centering mechanism. Handle 18 in FIG. 4B includes an opening 140 configured to receive a shaft 120, shown in FIG. 4A. As shown in FIGS. 4A-B, at least one portion of a proximal region of shaft 120 may include angled surfaces that engage with complimentary angled surfaces on a mating region of handle 18. The complimentary surfaces may engage with each other as handle 18 attaches to shaft 120, consistently orienting handle 18 on shaft 120 in a set position relative to base 19 in FIG. 1.

For instance, a self-centering mechanism may comprise shaft 120 having a semi-circular distal region, including a flattened surface 121. Handle 18 may include a complementary flattened surface 119 configured to engage with flattened surface 121 on shaft 120. Aligning flattened surface 121 on shaft 120 with flattened surface 119 of handle opening 140 orients handle 18 in a set position relative to the longitudinal axis of base 19 shown in FIG. 1.

Handle 18 can also include a tapered indent 123 on a portion of handle 18 configured to mate with shaft 120. indent 123 on handle 18 can be configured to complimentarily mate with tapered protrusion portion 124 on shaft 120. Engaging the complimentary surfaces of tapered portion 124 on shaft 120 and indent 123 on handle 18 can orient handle 18 relative to shaft 120 in a set position.

Handle opening 140 can align with a threaded opening 117 in thumb screw 154, shown as protruding out from side apertures of handle 18. When complementary angled surfaces 119, 123 of handle 18 align and mate with complementary angled surfaces 121, 124 on shaft 120, a threaded fastening portion 115 on a distal region of shaft 120 can pass through handle opening 140 and into a threaded opening 117 in thumb screw 154. Once handle 18 is aligned on shaft 120, a user can then rotate thumb screw 154 to fasten handle 18 in place, and threaded opening 117 of thumb screw 154 mechanically fastens handle 18 to shaft 120. Accordingly, the complimentary angled surfaces on the mating regions of handle 18 and shaft 120 can act as a self-centering mechanism, mating handle 18 to shaft 120 in a set, predetermined, orientation relative to base 19.

In other embodiments, handle 18 may be able to mate and lock into place on shaft 20 in more than one orientation relative to base 19. In FIG. 2, handle 18 is shown attached to shaft 20 so that handle 18 is not directly parallel with the longitudinal axis of base 19, or "off-axis." In certain embodiments, a user may attach handle 18 to shaft 20 in a number of different positions relative to base 19, so as to provide the most comfort for the user. For instance, handle 18 may lock onto shaft 20 so as to extend parallel with the longitudinal axis of base 19, or handle 18 may be rotated around the axis of shaft 20 to lock handle 18 into other discrete positions that are off-axis with base 19. In some embodiments, handle 18 may be capable of locking in two or more distinct positions on shaft 20 relative to base 19.

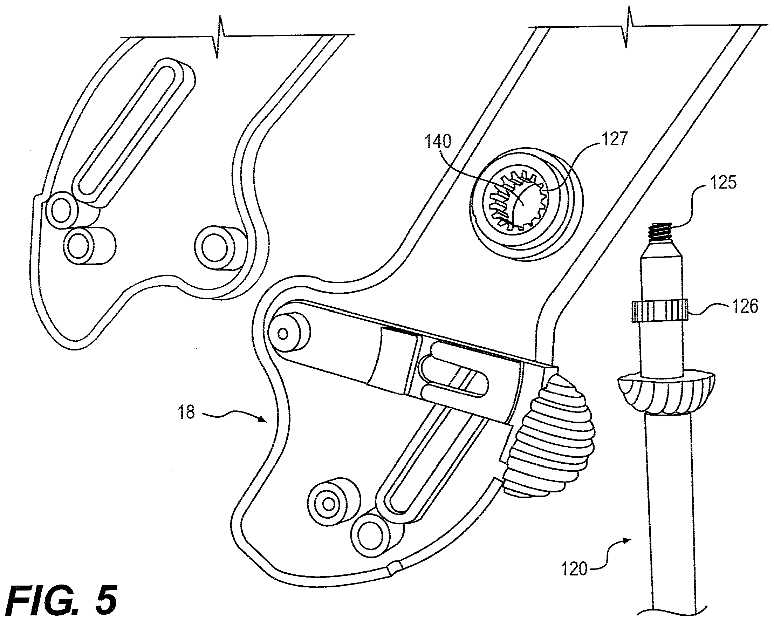

To allow a user to lock handle 18 into position on shaft 20 in more than one orientation relative to shaft 20 and base 19, a proximal region of shaft 20 can include angled surfaces capable of engaging with angled surfaces on handle 18 at more than one orientation. As is shown in FIG. 5, shaft 120 may include a spline region 126 complimentary to a female spline region 127 located around the circumference of an opening 140 in handle 18. Thus, handle 18 and a proximal mating region of shaft 120 can include a number of complimentary angled surfaces configured to engage with each other at multiple possible discrete angles relative to base 19. In this embodiment, the symmetry of the angled portions of complimentary spline regions 126 and 127 can allow handle 18 to mate with shaft 120 at more than one discrete angle, while still centering handle 18 on shaft 120 and locking handle 18 into place in set locations relative to base 19.

Once aligned, a threaded region 125 on shaft 120 can pass through handle aperture 140 and can engage with a threaded section of a thumb screw (not shown). A user can then rotate the thumb screw so as to fasten handle 18 to shaft 120. Accordingly, complimentary spline regions 126 and 127 on the mating regions of handle 18 and shaft 120 can act as a self-centering mechanism to position handle 18 on shaft 120 at multiple possible angles relative to base 19.

In an ambidextrous embodiment of handle 18, both sides of handle 18 may have apertures 140 including angled mating surfaces like 123, 119, 127. Additionally, handle 18 may have any number, arrangement, or shape of angled surfaces configured to be complimentary to angled surfaces on mating regions of shaft 120. For instance, the angled surfaces could be flat, pointed, rounded, or irregular. The angled surfaces of handle 18 and their complimentary angled surfaces on shaft 120 could project inwards or outwards from the face of handle 18, or they may project into or out of opening 140.

As discussed above and shown in FIGS. 7A-C, for example, an attachment mechanism 202 may include a thumb screw 254 for mating with a threaded shaft (not shown). In addition to attachment mechanism 202, various other control elements 40 may be used to attach handle 18 to shaft 20. Thumb screw 254 may be positioned in an interior, center region of handle 218 and may project out of handle apertures 232, 234, as depicted in FIGS. 7A-7C. An aperture in thumb screw 254 may align with an opening 240 in handle 218, through which the threaded shaft may pass. Thumb screw 254 may span the entire width of handle 218. Opening 240 may extend across at least part of the entire height of handle 218.

In other embodiments, thumb screw 254 may not span the entire width of handle 218. If opening 240 in handle 218 and thumb screw 254 do not span the entire width of handle 218, then the threaded shaft may mate with an opening on either side of handle 218. It is also contemplated that a lever, a slide, or other attachment mechanism 202 could removably couple handle 18 to shaft 20.

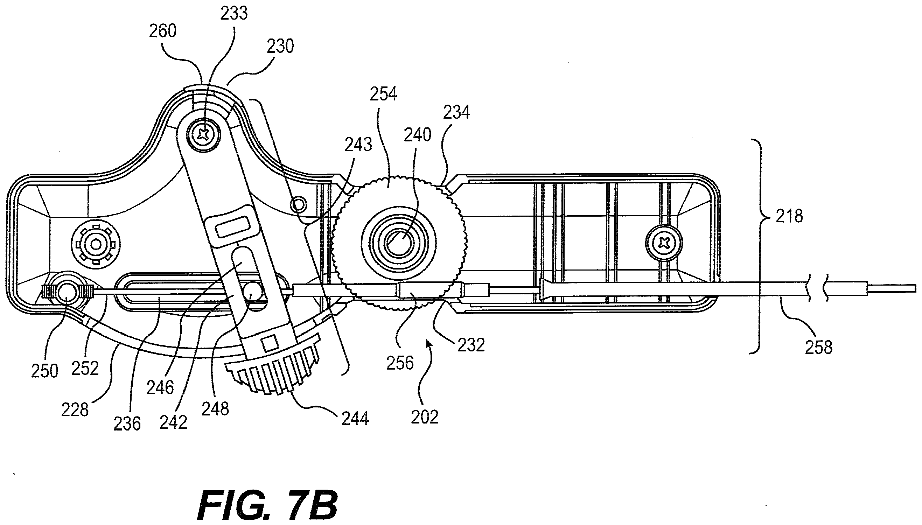

As shown in FIG. 7B-C, thumb screw 254 can protrude out from the side walls of handle 218 through apertures 232, 234. A user can access thumb screw 254 to rotate thumb screw 254 in either a clockwise or counter-clockwise direction to attach or detach handle 218 from the threaded shaft. To facilitate use, thumb screw 254 can protrude from both sides of handle 218 to provide the user with access to both sides of thumb screw 254 both when handle 218 is configured in a right-handed or a left-handed orientation on the shaft. Rotating thumb screw 254 in the same direction can fasten handle 218 to the shaft in both right-handed and left-handed configurations to facilitate ambidextrous use.

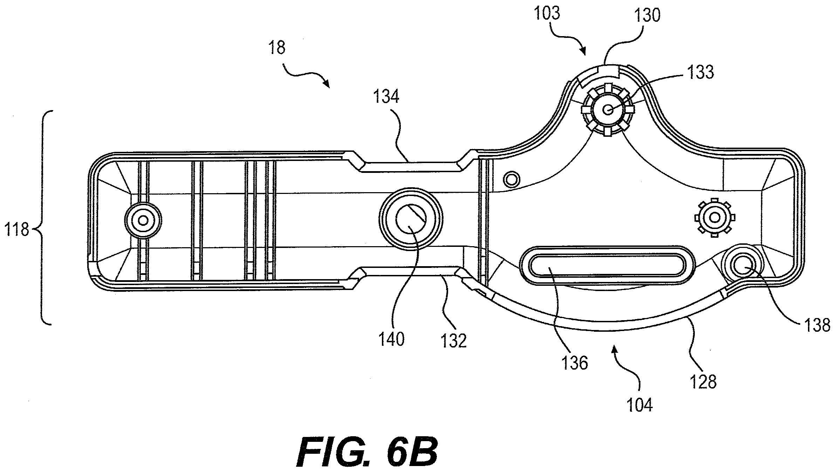

Handle 18 in FIG. 1 can also be configured for fixed attachment to shaft 20. In such a configuration, apertures 134, 132 in handle 18 may include at least one insert element 50 (not shown) configured for placement within apertures 134, 132 (FIG. 6B). In this embodiment, handle 18 can either be non-moveably fixed or moveably fixed to shaft 20. If handle 18 is non-moveably fixed, it may not be moved relative to shaft 20 and may only mate with shaft 20 in one position.

Alternatively, if handle 18 is moveably fixed, it may be moved relative to shaft 20, but cannot be completely detached from shaft 20. For instance, handle 18 may be able to shift upwards to a more proximal region of shaft 20, then rotate or slide once in the upwards position, and then shift back downwards in order to lock into place in a new orientation on shaft 20. For instance, handle 18 may include a spring to bias handle 18 down onto shaft 20. A user could apply force to handle 18 and move handle 18 upwards on shaft 20, rotate handle 18 on shaft 20 to reposition it, and then release the force on handle 18, allowing the spring to pull handle 18 back down further onto shaft 20. Handle 18 may include a number of other control elements 40 configured to moveably and fixedly couple handle 18 to shaft 20. For instance, a portion of handle 18 could extend into shaft 20 and include a push pin (not shown) to allow handle 18 to shift relative to openings in shaft 20.

To facilitate removal and orientation in a fully detachable embodiment, handle 218 may include markings 227 indicating the handedness of handle 218, as shown in FIG 7A. For instance, at least one side of handle 218 may include righthand or left-hand marking 227 indicating that when this marked side is oriented towards the user, handle 218 is connected to the shaft so as to facilitate right-handed or left-handed use, respectively. Additionally, handle 218 may include marking 225 indicating how to attach or detach handle 218 from shaft 20 (shown in FIG. 1). For instance, if handle 218 was held in place on a threaded shaft 20 via thumb screw 254, handle 218 may include marking 225 indicating which way to rotate thumb screw 254 in order to release handle 218 from threaded shaft 20. Handle 218 may include one or multiple of markings 227, 225 on one or multiple sides of handle 218.

As shown in FIG. 6B, handle 18 can include two additional apertures 128, 130 located on opposite walls of handle 18. In this embodiment, apertures 128, 130 extend along opposing side walls of handle 18. A first aperture 130 can be located on a protrusion 103, extending perpendicularly out from a longitudinal axis 102 (shown in FIG. 6A) of handle 18. A second aperture 128 is located on an opposite portion 104 of handle 18, projecting out from longitudinal axis 102 of handle 18. A user can place a thumb on portion 104 and place an index or middle finger on protrusion 103. The user can grasp the narrow center proximal portion of handle 18 with the remaining fingers. This and other configurations of handle 18 are possible.

One or more apertures 128, 130 on handle 18 can be configured to receive either control element 40 for controlling a function of device 10 or insert element 50 for at least partially covering aperture 128, 130. Aperture 128, 130 can thus be configured to receive control element 40, or instead, to be replaced with insert element 50, depending on which configuration of handle 18 should be manufactured. Each different configuration of handle 18 can include a common chassis and various control elements 40 or insert elements 50.

FIGS. 7A-9C show handle 18 of controller 12 in three possible operational configurations. In FIGS. 7A-C, one aperture 228 of handle 218 is shown receiving a first type of control element 243, while aperture 230 is shown receiving a first type of insert element 260. Thus, handle 218 can be configured to include control element 243 and insert element 260. This configuration could be used, for instance, with end-effector 16 requiring only one type of actuation, for instance opening and closing.

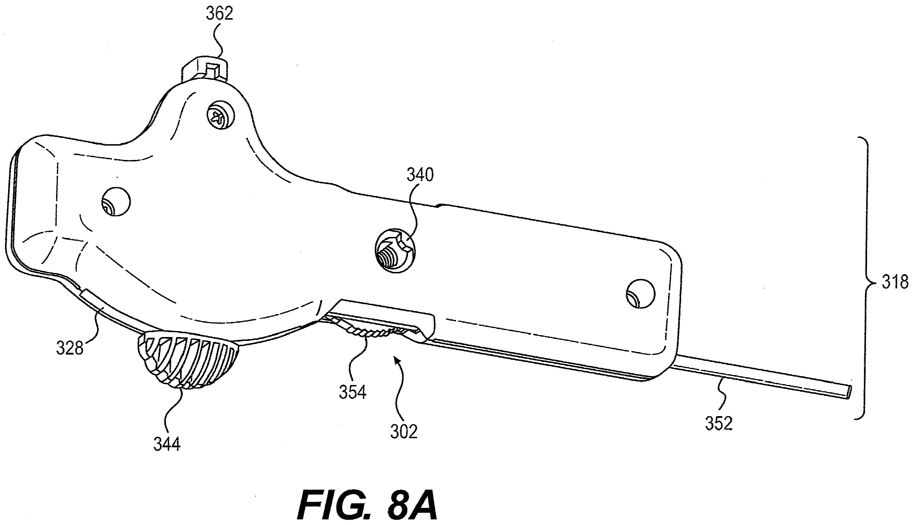

In contrast to FIGS. 7A-C, FIGS. 8A-C show both apertures 328, 330 receiving control elements 343, 363. Thus, handle 318 is shown with two control elements 343, 363. This configuration could be used, for instance, with end-effector 16 requiring two types of actuation, for instance opening and closing and remaining open or closed.

FIGS. 9A-C show a third configuration of handle 418 in which both apertures 428, 430 receive insert elements 443, 460. Thus, handle 418 is shown with no control elements 40 for controlling actuation. Only control element 402 is shown for controlling attachment of handle 418 to shaft 20. This configuration could be used, for instance, with end-effector 16 requiring no actuation, for instance a suction/irrigation tube. All three handle configurations shown in FIGS. 7A-9C can be achieved using the same basic handle chassis, shown in FIGS. 6A-B, having the same apertures 128, 228, 328, 428 and 130, 230, 330, 430.

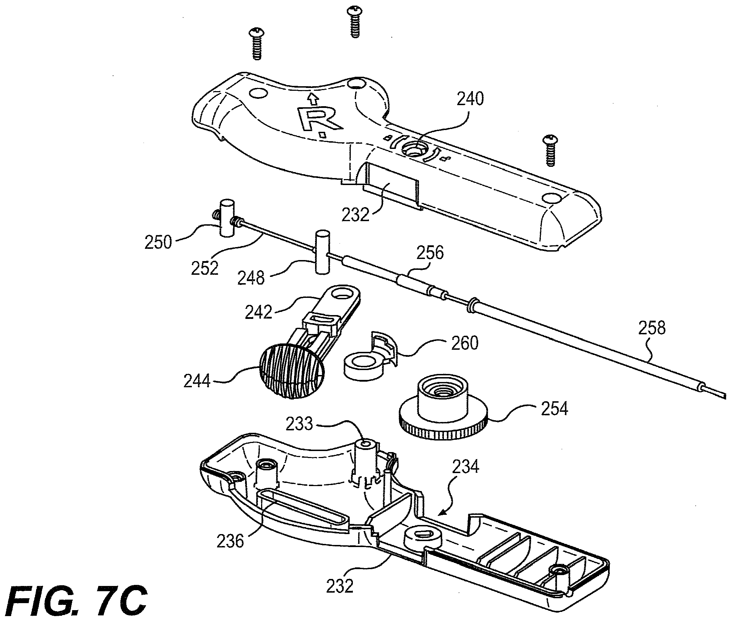

An aperture or opening of controller 20 can be configured to receive a variety of suitable control elements 40. In FIGS. 7A-C, control element 243 comprises an actuation trigger. Trigger 243 can actuate end-effector 16 via movement of a control member 252. As shown in FIG. 7B, trigger 243 can include a lever 242 and a finger grip 244 configured for manipulation by a user. As shown in FIGS. 7A and 7C, actuation lever 242 can extend from an interior region of handle 218, through handle aperture 228, and to a location exterior of handle 218. Grip 244 can be located on an end region of actuation lever 242 and can project out from aperture 228 in handle 218.

Actuation lever 242 can be rotationally coupled to handle 218 and can be configured to move control member 252 proximally and distally inside handle 218. As shown in FIG. 7B, actuation lever 242 can rotate around a pivot point 233, located in an interior region of handle 218 opposite from the side of handle 218 from which finger grip 244 protrudes. Actuation lever 242 can rotate when a user moves grip 244 from a proximal end to a distal end (or vice versa) of aperture 228. Actuation lever 242 can also include an opening 246 extending through a middle region of lever 242 located in handle 218. Pin 248 can be configured to engage opening 246 in lever 242. In FIG. 7B, pin 248 can extend through opening 246 in lever 242. Pin 248 can also be operatively connected to a control member 252, which may include an actuation cable.

Actuation cable 252 can also be attached to a second distal pin 250, as shown in FIGS. 7B-C. Cable 252 can extend longitudinally through handle 218 from distal pin 250 to proximal pin 248 (extending through lever opening 246), continue along a length of handle 218, and exit a proximal region of handle 218. As shown in FIG. 1, actuation control member 22 can exit handle 18 and continue to base 19, wherein slack in member 22 is shown between the proximal ends of handle 18 and base 19. Member 22 can also continue through base 19, extend through elongate shaft 14, and be operatively coupled to distal end-effector 16.

Referring to FIG. 7B, when a user engages lever 242 by sliding grip 244 proximally or distally along handle aperture 228, lever 242 can rotate around pivot point 233. As lever 242 pivots, lever opening 246 can engage proximal pin 248 and force proximal pin 248 towards or away from distal pin 250. The movement of unanchored pin 248 relative to anchored pin 250 can tension actuation cable control member 252. This tensioning force can be transmitted along control member 252, through instrument shaft 14, and to a distal region of instrument 10, where it can actuate end-effector 16. As such, trigger control element 243 of FIGS. 7A-C can actuate end-effector 16 of FIG. 1.

Actuation cable control member 252 in FIGS. 7A-C can include a Bowden cable. Distal pin 250 can be operatively connected to an inner portion of Bowden cable 252, and proximal pin 248 can be operatively connected to an outer portion of Bowden cable 252. When proximal pin 248 is forced towards or away from distal pin 250 by trigger lever 242, the outer portion of Bowden cable 252 can be lengthened or shortened relative to the inner portion, thus actuating end-effector 16 in FIG. 1.

In FIG. 8B, trigger lever element 343 can be located in aperture 328 opposite aperture 330, which can be configured to receive another control element 40, trigger release element 363. In this embodiment, trigger release element 363 can include a trigger release button 362. In FIG. 8A, trigger release button 362 of trigger release element 363 can protrude out of aperture 330 from an area inside handle 318. Trigger release button 362 can be moveably coupled to handle 318. For instance, trigger release button 362 may be rotationally, depressably, or slidingly coupled to handle 318.

As shown in FIG. 8B, trigger release element 363 can extend through aperture 330 and control element 343. In this embodiment, trigger release element 363 located in aperture 330 and actuation trigger lever 342 located in aperture 328 can be operatively coupled.

In the embodiment shown, trigger release element 363 can include a ratchet 370. Ratchet 370 can rotate around a pivot point 371. As shown in FIG. 8C, ratchet 370 can be positioned inside handle 318 between a spring 366 and a trigger release element 363. Trigger release element 363 can include trigger release button 362, which can extend out of aperture 330 and be configured for user manipulation, and a distal, oval bearing surface 364. Bearing surface 364 can be configured to encircle a pivot point 333 of actuation lever 342. Trigger release button 362 and oval bearing surface 364 of trigger release element 363 may be formed of one piece, or trigger release element 363 may be formed of separate pieces.

A region of oval bearing surface 364 can contact ratchet 370. In FIG. 8C, the rectangular protrusion on ratchet 370 facing trigger release element 363 can contact a region of bearing surface 364 of trigger release element 363. In other embodiments, ratchet 370 may directly or indirectly contact any portion of trigger release element 363. The opposite side of ratchet 370 can contact spring 366. As shown in FIG. 8C, ratchet 370 can contact spring 366 in a circular receiving section of ratchet 370. In other embodiments, any portion of ratchet 370 may directly or indirectly contact any portion of spring 366.

Actuation lever 342 can be positioned across spring 366, ratchet 370, and a portion of trigger release element 363, as shown in FIG. 8B. Spring 366 can sit against well 336 and push on ratchet 370, biasing ratchet 370 towards trigger release element 363. Ratchet 370 can have an arch-shaped opening 373 extending through its center region. Along at least a portion of the perimeter of opening 373 can extend a series of ratchet teeth 374. In this embodiment, ratchet teeth 374 can be shaped to permit one-way movement.

Actuation lever 342 can include an extension that protrudes into opening 373 in ratchet 370. This lever extension (not shown) can be configured to contact a portion of ratchet teeth 374. As spring 366 exerts force on ratchet 370, pushing ratchet 370 towards actuation lever pivot point 333, the extension on lever 342 can catch on teeth 374 of ratchet 370. When a user engages a grip 344 and rotates lever 342 in a proximal direction, the extension portion of actuation lever 342 can pass across the less steep slopes of ratchet teeth 374. If the user stops applying a force to actuation lever grip 344, the steep backwards slopes of ratchet teeth 374 can prevent the lever extension on lever 342 from passing backwards across ratchet teeth 374. As a result, the lever extension can remain in place, holding lever 342 in place, and trapping a proximal pin 348 in a lever opening 346 in an actuating position. Removing a force to lever grip 344 can cause lever 342 to stay in place and end-effector 16 (FIG. 1) to remain in a fixed position. This configuration allows a user to remove their hands from controller 12 without affecting the opening or closing of end-effector 16.

Trigger release element 363 can also be configured to allow actuation lever 342 to rotate freely about pivot point 333, allowing the user to move grip 344 freely back and forth in aperture 328 without engaging ratchet 370.

Oval bearing surface 364 of trigger release element 363 can be configured to allow axial movement around lever pivot point 333. Depressing trigger release button 362 can push oval bearing surface 364 further in an internal direction relative to handle 318. Because oval bearing surface 364 can contact ratchet 370, depressing button 362 can push ratchet 370 and force ratchet 370 to rotate around its pivot point 371 away from trigger release element 363. As ratchet 370 rotates away, it can compress spring 366. When ratchet 370 compresses spring 366 and shifts closer to grip portion 344 of lever 342, the extension on lever 342 no longer contacts teeth portion 374 of ratchet opening 373, and the extension on lever 342 can be released from the steep ratchet teeth slopes 374 that had locked trigger lever 342 in place. Accordingly, lever grip 344 may be freely rotated proximally and distally in aperture 328.

In another aspect, a user can rotate trigger release element 363 around pivot point 333 by moving trigger release button 362 proximally or distally within aperture 330. In some aspects of the invention, rotating trigger release button 362 may exert or release force on ratchet 370 through contact with oval bearing surface 364 of trigger release element 363, causing ratchet 370 to rotate, and thereby moving actuation lever 342 from a ratcheting to a freely moving configuration.

One of skill in the art will appreciate that although two apertures are discussed with regard to the embodiments shown in FIGS. 7A-9C, handle 18 could be configured to include any number of apertures; for instance, one, three, four, or more apertures could be located on device 10. In addition, while the apertures of this embodiment appear on two opposing side walls of handle 18, apertures 128, 130 may appear on any face of handle 18. For instance, one or more apertures 128, 130 could appear on a distal wall, a proximal wall, a top face, or a bottom face of handle 18.

In addition, apertures 128, 130 may be any size or shape in addition to the sizes and shapes depicted in these embodiments. Apertures 128, 130 may be rectangular, square, circular, oval, irregularly shaped, etc. In addition, apertures 128, 130 configured to receive either control element 40, or interchangeably insert element 50, can be located anywhere on controller 12 or anywhere on device 10.

Multiple configurations of various elements on device 10 are not limited to those described above for handle 18. For example, another portion of device 10 can include aperture 128 configured to receive control element 40 or insert element 50. Base 19 of controller 12, shaft 20, elongate shaft 14, or end-effector 16, for instance, can include aperture 128, 130 configured to receive either control elements 40 or insert elements 50, interchangeably.

As described above, device 10 may also be configured for enhanced electrical safety. In one embodiment, device 10 can be configured to protect a user from stray electrical current. For example, handle 18 may be formed so as to minimize the number of open air gaps that could otherwise allow stray current to travel along device 10 and shock the user. For instance, one or more apertures in controller 12 could either receive control elements 40, such as an actuation trigger lever or trigger release button, or otherwise be replaced with insert elements 50 to substantially cover the apertures, as described above. This can minimize air gaps in controller 12, reducing the likelihood of an electrical shock traveling to the user.

Electrical safety can be also be enhanced by using non-conductive material in device 10. For instance, all or part of base 19, shaft 20, or handle 18 may be formed of non-conductive material. In addition, control elements 40 or insert elements 50 configured for positioning in any handle apertures may be formed of non-conductive material. In one embodiment, controller 12 can be formed substantially of non-conductive material. Various non-conductive materials may be used depending upon the operational and structural requirements of device 10 or its components.

In some embodiments, both hot and cold instruments alike are formed of non-conductive components or insulated components. Accordingly, if electrical energy were accidently transferred to a cold instrument through contact with a hot instrument, through capacitive coupling, or through some other method of transfer, the insulation would protect the cold instrument as if the instrument were a hot instrument. Aspects of the present invention could provide an added layer of protection for cold instruments using passive electrical conductors, and reduce risks associated with stray voltages or currents. Accordingly, both actively conductive and passively conductive devices can be formed of non-conductive or insulating material, or grounded.

In other embodiments, parts in the elongate shaft 14 of device 10, or parts in the distal end-effector 16 can be formed of non-conductive material. For instance, elongate shaft 14 of device 10 may be formed of a plurality of articulation segments configured to provide instrument steering. These segments may be formed of non-conductive material. In addition, distal end-effector 16 can be formed of non-conductive material. Further, cable guides, screws, or any other mechanical parts included in device 10 can be formed of non-conductive material, further preventing unwanted current from traveling through device 10 and injuring the patient or the user.

In other embodiments, it may not be possible or desirable to form some parts of device 10 of non-conductive material. Alternatively, it may be possible to insulate the conductive parts with non-conductive material. For instance, an actuation control member, which could include a cable, a wire, a ribbon, a rod, or other elongated control member in device 10, may include insulating material. Control members may have insulation over a portion of their length, over substantially their entire length, or the control members may have multiple layers of insulation covering a portion or substantially their entire length.

For example, FIG. 7B shows control member 252, housed within controller handle 218 with two separate sections of insulation 256, 258. Insulation 256 can be located adjacent to aperture 232 to reduce the likelihood that an electrical shock discharges to the user. Insulation 258 could extend through a proximal region of handle 218 to reduce the likelihood of shocking by direct contact between the user and the conductive member.

Parts of elongate shaft 14 of device 10 (shown in FIG. 1) may include conductive material. For instance, elongate shaft 14 can include a rigid proximal section 24 and a flexible distal section 26. Rigid proximal shaft portion 24 can be made of either non-conductive material, or alternatively, can be made of conductive material, for instance stainless steel. If rigid proximal shaft portion 24 is made of a conductive material, it can be covered in insulating material, isolated from other conductive components, or grounded. Flexible distal section 26 may include various types of elastically flexible materials.

In certain embodiments, one or more components of device 10 may contain a conductive element, such as, for instance, an actuation member. As shown in Fig. 7B, control member 252 may be connected to control element 243 inserted in aperture 228 of handle 218. Control member 252 may be formed, at least in part, of conductive material. For instance, control member 252 can include a control cable that can be formed of conductive material, for instance stainless steel.

Alternatively, control member 252 can be formed of non-conductive material. Such non-conductive materials may have properties, like tensile strength and modulus, substantially similar to those of steel cables. Other non-conductive materials suitable for forming control member 252, for instance, can include thermoplastic polyethylene, such as ultra-high molecular weight polyethylene, or polyamides, such as Kevlar.

In embodiments of device 10 containing conductive components, the conductive components may be isolated so that unwanted electricity does not readily travel along device 10 into controller 12 and shock a user. For instance, device 10 may include conductive structural components, such as braided mesh, articulation segments, rods, or control members to help support and guide elongate shaft 14 of device 10. If these structural components come in contact with each other or with a component that extends along a substantial length of device 10, such as a control member, then they may form a continuous path for unwanted electricity to flow along. Accordingly, some embodiments may try to create a gap between the various conductive elements contained in device 10 in order to interrupt the flow of unwanted current.

For instance, conductive components may include abraded surfaces in order to minimize contact with surrounding conductive surfaces. In one embodiment, the proximal region of a control member may be abraded where it enters controller 12. In other embodiments, the proximal region of a control member may include a non-conductive buffer, seal, or end cap. In still other embodiments other regions of a control member, or a mesh braid or other conductive component in elongate shaft 14 may be abraded, sealed, or otherwise isolated so as to prevent it from contacting a control member or other component that may extend the length of device 10. Accordingly, isolating conductive components in device 10 would help prevent unwanted electricity from traveling along a control member or other conductive component, through the length of device 10, and to a user.

Electrically powered instruments may be used in conjunction with an endoscopic system, such as that shown in FIG. 2. In FIG. 2, two devices 10, 10' are shown flanking a center space 3 configured for a user. Device 10 can include a "hot" instrument configured to provide electrical energy to end-effector 16 or a distal end of shaft 14. The hot instrument can be used alone, with other electrical instruments, or with "cold" instruments. The cold instruments could include one or more conductive members. Conductive members as referred to here can be actively or passively conductive. While these conductive members may not be designed to actively transmit electrical energy, they could if touched to a hot instrument or other source of electrical energy.

Traditional laparoscopic systems introduce multiple instruments through a single port, whereby rigid instruments typically cross each other at that single entrance. The instruments form an X-shaped relationship between adjacent instruments, pivoting relative to each other at the point. In certain embodiments, one of more instruments (including a hot instrument) may be inserted through one or more channels in guide tube 17. These instruments may run parallel to each other for the length of guide tube 17, which may extend longitudinally for a distance of at least one foot. In some embodiments, the flexible region of guide tube 17 may be between one to two feet in length, and the flexible distal section 26 of elongate shaft 14 may be at least two feet long.

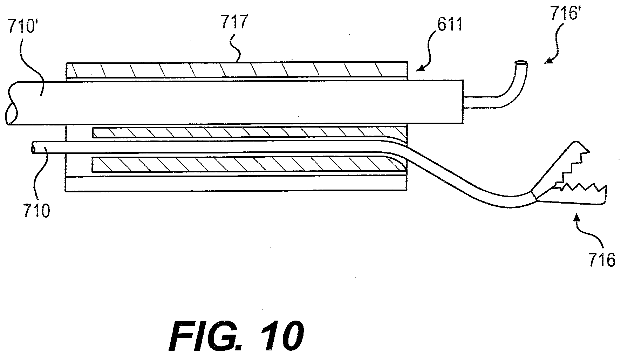

Close proximity of hot and cold instruments can create a safety concern because of the close parallel relationship of the two instruments. As shown in Fig. 10, guide tube 717 can include one or more channels 611. Channels 611 may be diverging, converging, or parallel in orientation relative to each other. Devices 710, 710' may be located in channels 611 and extend from a distal region of a guide tube 717 in diverging, converging, or parallel orientation relative to each other. Embodiments of the present invention may improve electrical safety for instruments in generally close proximity and in generally parallels configurations.

Additionally, conductive components of device 10 can be grounded, providing any unwanted electrical current with a common return path away from the patient, the device, and the user. Grounding can be used with conductive components instead of insulation, or in addition to insulation in order to add an extra layer of protection in the event of insulation failure. For instance, portions of control members may be both covered with insulation and grounded. In one embodiment, controller base 19 can contain at least one grounded conductive member, for instance, an insulated conductive actuation cable, to drain unwanted electrical current away from the instrument or the user.

Other aspects of the invention may promote electrical safety by limiting the ingress of fluids and external materials, like blood, mucus, feces, or tissue, into device 10 or system 15. A user may need to insert the endoscopy system into a wet environment, for instance a surgical site. Because body fluids, saline solution, and patient tissue can conduct electricity, preventing these from entering device 10 or guide tube 17 can enhance electrical safety by eliminating unintended transfers or buildup of voltage. Accordingly, certain embodiments of the present invention are designed to deter the ingress of liquids. This can be achieved through the use of seals or covering materials that are substantially impermeable to liquids.

For instance, actuation or articulation cables, wires, ribbons, elongate structures, pulleys, gears (collectively, control members) inside device 10 and guide tube 17 can be formed of substantially liquid impermeable materials. If liquid does enter device 10 or guide tube 17, the control members can be substantially kept out of contact with fluid, helping prevent electrical current from traveling along the control members and into controller 12, potentially shocking a user. Additionally, fluid buildup in and around control members may inhibit the instrument's ability to articulate and actuate. For instance, blood or other fluids may coagulate on the control members, clogging the instrument and reducing its range of motion.

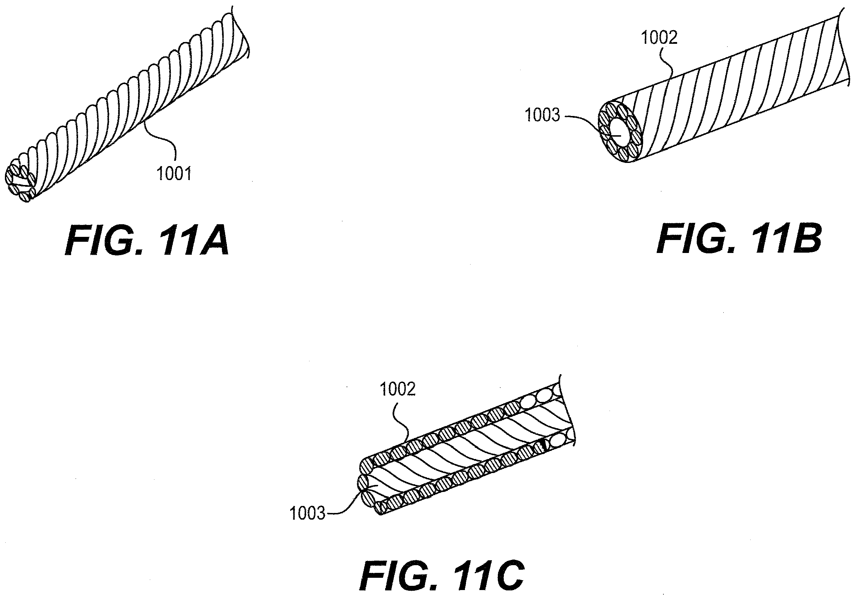

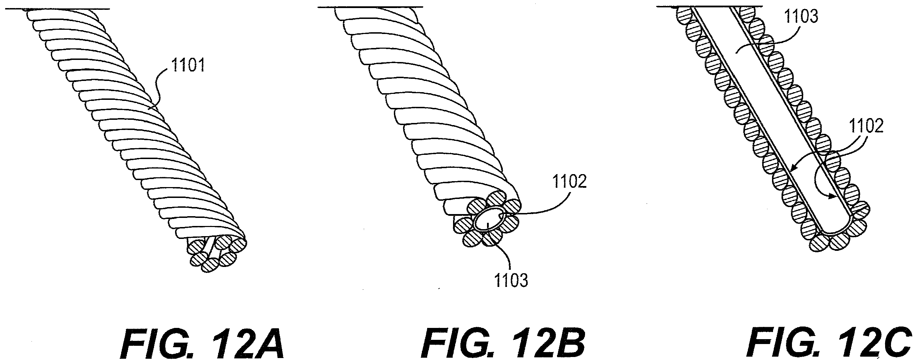

In one embodiment, sealed Bowden cables, as shown in FIGS. 11A-C and 12A-C, are used to help prevent fluid from reaching the control cables of the device or guide tube. For instance, Bowden cables can be used in the present invention as articulation cables, actuation cables, or both. Bowden cables can also be used to surround other control wires, cables, or control members. Accordingly, Bowden cables can be used on their own, or in conjunction with other control members. Bowden cables can be used in controller 12, elongate shaft 14, end-effector 16, guide tube 17, or anywhere in surgical system 15.

In FIGS. 11A-C, a sealant 1002 on a Bowden cable 1001 bridges gaps between the helical wires of Bowden cable 1001, substantially preventing fluid from wicking through the wires and into a central control cable space 1003. Sealant 1002 can be used on the inner or outer surfaces of Bowden cable 1001, or to seal the ends of Bowden cable 1001. One of ordinary skill in the art will appreciate that a variety of sealants can be used to create sealed Bowden cables, for instance silicone, urethane, Teflon, or other suitable polymers or materials.

Another aspect of this disclosure pertains to the manufacture of sealed Bowden cables. In one embodiment, a Bowden cable is assembled and then sealed by mixing a low-durometer, flexible sealant and a solvent to create a low-viscosity solution. The low viscosity solution can allow for sufficient flow and deposition of the sealant between the wires of the Bowden cable. The solution can then be applied to the outer surface of the Bowden cable, and the solvent can be allowed to evaporate, at which point the sealant can return to its natural, ductile state. Alternatively, the solution may be applied to the inner surface of the Bowden cable, or to both the outer and inner surfaces. This method uses a solvent vehicle to decrease the viscosity of the resulting sealant solution to spread the sealant between the Bowden cable wires, forming a superior seal to prior art methods. This method results in a flexible film that fills the gaps between the wires, creating a sealed Bowden cable that is substantially impermeable to fluid.

Various different sealants and solvents may be appropriate for use in the above method. For instance, the sealant can include silicone or urethane, or other suitable polymers. The solvent can include Methyl Ethyl Ketone (MET) or toluene, for instance.

In another embodiment shown in FIGS. 12A-C, a sealant 1102 can be press fit onto the inner surface of a Bowden cable 1101 to help prevent fluid from entering a central control cable space 1103 of Bowden cable 1101. Alternatively, sealant 1102 can be applied to the outer surface of Bowden cable 1101, or to both the outer and inner surfaces. A number of different sealants may be appropriate for press fitting. The sealant can include a silicone extrusion, a urethane extrusion, or any appropriate polymer extrusion, for instance. In one embodiment, the extrusion is applied before instrument assembly.

In another embodiment, one or more liners can be used on the inner surface, outer surface, or both the inner and outer surfaces of a Bowden cable. One of ordinary skill in the art will recognize that a number of different materials would be appropriate for use as a liner, for instance, Teflon. In yet another embodiment, a grease, for instance, a silicon grease, can be applied to the outer or inner surface to seal the Bowden cable.

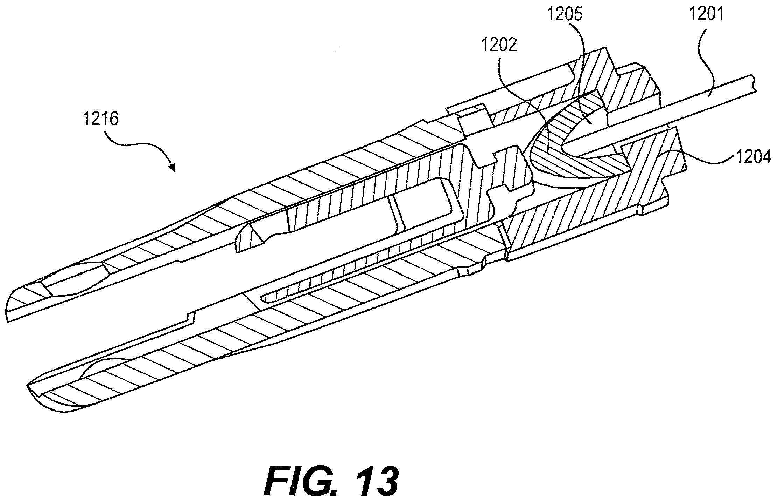

In another embodiment, the space around the control members can be substantially sealed to help prevent the ingress of fluid into the control member area. A hollow, dome-shaped seal 1202 can be fitted around a control member 1201, as shown in FIG. 13. Control member 1201 can be a Bowden cable or other control cable or wire, an insulated or non-insulated member, or a member sealed or not sealed according to the above methods.

The narrow top of dome seal 1202 can encircle control member 1201, while the wide bottom of dome seal 1202 can press against a wall 1204 through which control member 1201 runs. The close fit between dome seal 1202 and control member 1201, and dome seal 1202 and wall 1204 can at least partially block liquid from entering an inner seal space 1205 housing control member 1201. The hollow, dome shape can create inner seal space 1205 between control member 1201 and wall 1204 so that dome seal 1202 can elastically deform when control member 1201 is tensioned, allowing for appropriate tensioning of control member 1201 in response to user input. This arrangement allows control member 1201 to move freely during articulation or actuation. In one embodiment, the wide bottom of dome seal 1202 can fit snugly with interior wall 1204 of a distal end-effector 1216, as shown in FIG. 13, through which the distal end of control member 1201 runs. This can prevent fluid that may enter distal end-effector 1216 from entering inner seal space 1205, through which member 2101 runs, and gaining access into device 10.

A number of materials may be appropriate for use in forming dome-shaped seal 1202. For instance, dome seal 1202 can include urethane, silicon, or any other low-durometer material.

In addition, other shapes that provide internal spacing as in FIG. 13 can be used to create a seal in accordance with the invention. For instance, a hollow square, rectangle, triangle, sphere, or cone-shaped seal could be used.

The various components of the medical device described herein may be made of a suitable biocompatible material and may be flexible, for example, to traverse tortuous anatomy in the body. Any aspect set forth in any embodiment may be used with any other embodiment set forth herein. Every device and apparatus set forth herein may be used in any suitable medical procedure, may be advanced through any suitable body lumen and body cavity, and may be used to visualize, acquire, treat, or remove tissue from any suitable body portion.

It will be apparent to those skilled in the art that various modifications and variations can be made in the disclosed systems and processes without departing from the scope of the invention. Other embodiments of the invention will be apparent to those skilled in the art from consideration of the specification and practice of the invention disclosed herein. It is intended that the specification and examples be considered as exemplary only, with a true scope of the invention being indicated by the following claims and their equivalents.

## Claims

1. A surgical instrument, comprising:
an elongate shaft (14) having a proximal end and a distal end;
a controller (12, 12') coupled to a proximal region of the elongate shaft (14), a control element (40), an end-effector (16, 16') and an insert element (50); the controller (12, 12') comprising an aperture (428, 430), wherein the controller (12, 12') in a first configuration is configured to control the end-effector (16, 16') coupled to the distal end of the elongate shaft (14) via the control element (40) moveably coupled to the controller (12, 12') and received by the aperture (428, 430) wherein in a second configuration the controller (12, 12') is configured to move the distal end of the elongate shaft (14) and the insert element (50) is fixedly coupled to the controller (12, 12') and configured to cover the aperture (428, 430).

2. The surgical instrument of claim 1, wherein the controller (12, 12') in the first configuration includes a set of control elements (40) and the controller (12, 12') in the second configuration includes a set of insert elements (50) different to the set of the control elements (40).

3. The surgical instrument of claim 1, wherein the control element (40) is at least one of rotatably and slideably coupled to the controller (12, 12').

4. The surgical instrument of claim 1, wherein the control element (40) and insert element (50) both have an outer surface configured to engage an inner surface of the aperture (428, 420).

5. The surgical instrument of claim 1, wherein the control element (40) includes at least one of a thumb wheel, a lever, a button, a ratchet, a control member, a gear, a crank, and a spring.

6. The surgical instrument of claim 1, wherein the insert element (50) has an outer surface conforming to an outer surface of the controller (12, 12').

## Patentansprüche

1. Chirurgisches Instrument, das aufweist:
einen länglichen Schaft (14) mit einem proximalen Ende und einem distalen Ende;
eine Steuerung (12, 12), die mit einem proximalen Bereich des länglichen Schafts (14), einem Steuerelement (40), einem Endeffektor (16, 16`) und einem Einführelement (50) gekoppelt ist; wobei die Steuerung (12, 12') eine Öffnung (428, 430) aufweist, wobei die Steuerung (12, 12') in einer ersten Konfiguration so konfiguriert ist, dass sie den Endeffektor (16, 16') steuert, der mit dem distalen Ende des länglichen Schafts (14) über das Steuerelement (40) gekoppelt ist, das mit der Steuerung (12, 12') beweglich gekoppelt und durch die Öffnung (428, 430) aufgenommen ist, wobei in einer zweiten Konfiguration die Steuerung (12, 12') so konfiguriert ist, dass sie das distale Ende des länglichen Schafts (14) bewegt und das Einführelement (50) mit der Steuerung (12, 12') fest gekoppelt und so konfiguriert ist, dass es die Öffnung (428, 430) abdeckt.

2. Chirurgisches Instrument nach Anspruch 1, wobei die Steuerung (12, 12') in der ersten Konfiguration einen Satz von Steuerelementen (40) aufweist und die Steuerung (12, 12') in der zweiten Konfiguration einen Satz von Einführelementen (50) aufweist, der sich vom Satz der Steuerelemente (40) unterscheidet.

3. Chirurgisches Instrument nach Anspruch 1, wobei das Steuerelement (40) mit der Steuerung (12, 12') drehbar und/oder verschiebbar gekoppelt ist.

4. Chirurgisches Instrument nach Anspruch 1, wobei das Steuerelement (40) und Einführelement (50) beide eine Außenfläche haben, die so konfiguriert ist, dass sie einen Eingriff mit einer Innenfläche der Öffnung (428, 430) herstellt.

5. Chirurgisches Instrument nach Anspruch 1, wobei das Steuerelement (40) ein Rändelrad, einen Hebel, eine Taste, eine Sperrklinke, ein Steuerbauteil, ein Zahnrad, eine Kurbel und/oder eine Feder aufweist.

6. Chirurgisches Instrument nach Anspruch 1, wobei das Einführelement (50) eine Außenfläche hat, die an eine Außenfläche der Steuerung (12, 12') angepasst ist.

## Revendications

1. Instrument chirurgical comprenant :
un arbre allongé (14) ayant une extrémité proximale et une extrémité distale ;
un organe de commande (12, 12') couplé à une région proximale de l'arbre allongé (14), un élément de commande (40), un effecteur terminal (16, 16') et un élément d'insert (50) ; l'organe de commande (12, 12') comprenant une ouverture (428, 430), dans lequel l'organe de commande (12, 12'), dans une première configuration, est configuré pour commander l'effecteur terminal (16, 16') couplé à l'extrémité distale de l'arbre allongé (14) via l'élément de commande (40) couplé de manière mobile à l'organe de commande (12, 12') et reçu par l'ouverture (428, 430), dans lequel, dans une seconde configuration, l'organe de commande (12, 12') est configuré pour déplacer l'extrémité distale de l'arbre allongé (14) et l'élément d'insert (50) est couplé de manière fixe à l'organe de commande (12, 12') et configuré pour recouvrir l'ouverture (428, 430).

2. Instrument chirurgical selon la revendication 1, dans lequel l'organe de commande (12, 12'), dans la première configuration, comprend un ensemble d'éléments de commande (40) et l'organe de commande (12, 12') dans la seconde configuration comprend un ensemble d'éléments d'insert (50) différent de l'ensemble d'éléments de commande (40).

3. Instrument chirurgical selon la revendication 1, dans lequel l'élément de commande (40) est couplé au moins selon l'une parmi une manière rotative et coulissante à l'organe de commande (12, 12').

4. Instrument chirurgical selon la revendication 1, dans lequel l'élément de commande (40) et l'élément d'insert (50) ont tous deux une surface externe configurée pour mettre en prise une surface interne de l'ouverture (428, 430).

5. Instrument chirurgical selon la revendication 1, dans lequel l'élément de commande (40) comprend au moins l'un parmi une molette, un levier, un bouton, un encliquetage, un élément de commande, un engrenage, une manivelle et un ressort.

6. Instrument chirurgical selon la revendication 1, dans lequel l'élément d'insert (50) a une surface externe se conformant à une surface externe de l'organe de commande (12, 12').
